# EUROPEAN PATENT SPECIFICATION

(11) **EP 0 906 275 B1**
(45) Date of publication and mention of the grant of the patent: **27.06.2001**
(21) Application number: 97928596.2
(22) Date of filing: 17.06.1997
(51) Int. Cl.: C07C 323/59, A61K 31/195, A61K 31/22, A61K 31/225

(54) **NEW FORMS OF ORGANIC SALTS OF N'N-DIACETYLCYSTINE**
NEUE FORMEN DER ORGANISCHEN SALZE DES N'N-DIACETYLCYSTINS
NOUVELLES FORMES D'UN SEL ORGANIQUE DE N'N-DIACETYLCYSTINE

(30) Priority: 18.06.1996 SE 9602416
(43) Date of publication of application: 07.04.1999
(73) Proprietor: AstraZeneca AB, 151 85 Södertälje (SE)
(72) Inventor: JAKUPOVIC, Edib, S-155 00 Nykvarn (SE); TENEBERG, Eric, S-125 51 Älvsjö (SE)
(86) International application number: SE9701069
(87) International publication number: WO9748679

(56) References cited:
- WO-A-93/11104

## Description

The present invention relates to new hydrates of salts of N,N'-diacetylcystine and to processes for the preparation thereof.

### Background of the Invention

N-acetyl-L-cysteine is a well-known compound which is used as a therapeutic agent against chronic obstructive pulmonary diseases and chronic bronchitis. Although the first patent was filed in 1964 (GB 954268), the mechanism of action of the compound has not been established. It is also known that the corresponding disulphide of N-acetyl-L-cysteine, i.e., N,N'-diacetyl-L-cystine, L-DiNAC, acts as a potent immunostimulator (SE patent application No. 9002067-8), showing an activity comparable to contemporary immunostimulants such as sodium diethyl dithiocarbamate or 2,2'-dithiobisethanol.

It has recently been found that certain salts of DiNAC with organic bases exhibit a favourable combination of non-hygroscopicity and crystallinity which permits the isolation and formulation of these salts in solid form. They have the advantages of ease of crystallisation, non-hygroscopicity and chemical stability, as well as the immunomodulating activity of DiNAC, and are thus medically useful. These salts are described in WO 93/11104.

Salts composed of an organic base and N,N'-diacetylcystine (DiNAC) are generally prepared by mixing DiNAC and the organic base, as defined above, each dissolved or dispersed in a solvent or solvent mixture. Solvents, such as water, alcohols, glycols, ketones, amides, sulphoxides or other polar solvents may be used; solvent mixtures may also be used. The salt either precipitates directly from the reaction mixture, or is obtained by the addition of a less polar solvent, by evaporation, or by lyophilisation. The reaction is performed at elevated temperature or room temperature, depending on the solubility in the medium. Alternatively, the salt can be prepared by oxidation of the appropriate N-acetyl cysteine salt in an aqueous or alcoholic solution, followed by precipitation as above. The oxidation may be effected either chemically, using, e.g., hydrogen peroxide or a halogen, or electrochemically.

The above methods provide the organic salts of DiNAC in anhydrous form.

### Disclosure of the invention

We have now prepared novel hydrates of certain salts of N,N'-diacetylcystine. The invention, in one aspect, provides hydrated salts of N,N'-diacetylcystine, having the formula 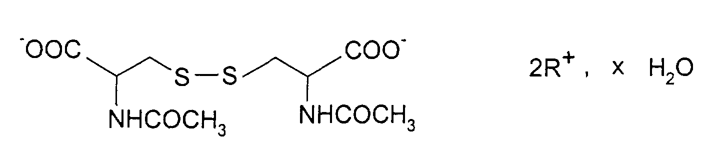 in which
the N,N'-diacetylcystine is the D-, L-, or meso form, or any mixture thereof,
R⁺ is the protonated form of lysine, and x is 2, designating the dihydrate.

The N,N'-diacetylcystine is preferably the L-form. The lysine is preferably L-lysine.

In another aspect, the invention provides a process for obtaining the hydrated salts. The process includes oxidising N-acetyl cysteine in the presence of lysine, or the protonated forms thereof, in an aqueous or alcoholic solution, preferably in the presence of a catalytic amount of an alkali metal hydroxide, and crystallising the hydrate using a solvent comprising ethanol.

Specifically, the hydrated forms of di-lysinium- N,N'-diacetylcystinate may be prepared for example by oxidising N-acetyl cysteine in an aqueous or alcoholic solution in the presence of lysine or a salt containing the protonated form of lysine, and a catalytic amount of an alkali metal hydroxide, and then to obtain the dihydrated salt, crystallising the product by adding ethanol to an aqueous solution thereof at a temperature of about 60°C or below, or by adding an aqueous solution of the product to a mixture of ethanol and water at a temperature of below about 60°C, for example at about 20°C or below.

The monohydrate form of the lysine salt of N,N'-diacetylcystine is metastable, while the dihydrate form is stable and especially suitable for use in inhalation devices.

By "catalytic amount" is meant a trace amount of alkali metal hydroxide; preferably at least 0.001 mole equivalent compared to the N-acetyl-L-cysteine is used, or up to 0.01 or 0.1 mole equivalent. Preferably no more than 0.5 mole equivalent is used. In one embodiment of the invention, about 0.1 mole equivalent of the alkali metal hydroxide is used.

Suitable alkali metal hydroxides are, e.g., sodium, potassium and lithium hydroxide.

The oxidation may be effected either chemically, using, e.g., hydrogen peroxide or halogen as oxidising agent, or electrochemically.

The hydrated salts of the present invention have immunomodulating activity and may be used for example in the treatment of diseases where an anergy of the immune response or an aberrant immune response or an ineffective host response can be suspected. Among such diseases are included the diseases listed in WO 93/11104, incorporated herein by reference. For example, diseases which may benefit from treatment with the present hydrated salts include chronic bronchitis, malignant diseases and chronic infections. The ability of the hydrated salts to modulate immune responses may be illustrated in the animal delayed type hypersensitivity (DTH) test in the mouse, as described in WO 93/11104. The present hydrated salts will also be useful in the treatment of chronic hepatitis B and/or C infections, as may be illustrated by a strengthening of the TH1-type response in the Leishmania model, as described for example by Connell et al ((N. Connell, E. Medina-Acosta, W. McMaster, B. Bloom and D. Russell, 1993, Effective immunisation against cutaneous leishmaniasis with recombinant bacilli Calmette-Guerin expressing the Leishmania surface proteinase p63, Proc. Natl. Acad. Sci. USA, 90:11473).

The new hydrated salts can be formulated for administration by inhalation, for example from a dry powder inhaler or from a pressurised metered dose inhaler (pMDI); alternatively, they can be formulated for oral, topical, or parenteral use. The formulations may include a pharmaceutically acceptable carrier.

The hydrated salts of the present invention can be included in different dosage forms, e.g., dry powders, aerosols, tablets, coated tablets, gelatine capsules and solutions.

For the preparation of a formulation for inhalation from a dry powder inhaler, the hydrated salts of the present invention may be combined with for example a pharmaceutically acceptable diluent or carrier and provided in the form of inhalable particles.

For the preparation of a formulation for inhalation from a pMDI the hydrated salts of the present invention may be dissolved or suspended in a suitable propellant optionally together with a co-solvent and/or one or more pharmaceutically acceptable surfactants or other excipients.

For the preparation of tablets, coated tablets and gelatine capsules the hydrated salts of the present invention can be combined with pharmaceutically acceptable materials, e.g., lactose, starch, dicalcium phosphate, microcrystalline cellulose, polyvinylpyrrolidone, gelatine, cellulose derivatives, colloidal silicone dioxide, talc and stearic acid or its salts.

For the preparation of oral solutions suitable excipients are water, saccharose, glucose, sorbitol, fructose and xylitol.

The dosage forms can besides mentioned excipients contain preservatives, stabilisers, viscosity regulating agents, emulsifiers, sweetening agents, colouring agents, flavouring agents, tonicity regulating agents, buffers or antioxidants. They can also contain other therapeutically valuable substances.

The invention is illustrated by the following non-limiting example.

### Example

### Di-L-lysinium-N,N'-diacetyl-L-cystinate dihydrate

N-acetyl-L-cysteine (50.0 g, 1.0 eq), L-lysine monohydrate (50.5 g, 1.0 eq), potassium hydroxide (1.6 g, 0.08 eq) and purified water (75 mL) were mixed and stirred until a clear solution was achieved. Hydrogen peroxide, (35% solution, 0.5 eq) was then added dropwise, with the temperature kept between about 20°C and about 40°C during the addition.
The dihydrate was then crystallised from the above solution, using each of the following methods:
1). The solution was added to 800 ml of ethanol at 60°C. After 3 h of stirring, the crystals were filtered off, giving 74 g (70%) of the title substance.
2). The solution was added to 800 ml of ethanol, containing 10% of water, at 20°C. After 10 h of stirring, the crystals were filtered off, giving 66.5 g (63%) of the title substance.
The ¹H-NMR and ¹³C-NMR were identical to those of the monohydrate.

Powder X-ray diffractograms of the product measured from 1 to 40° in 2θ show the following peaks, characteristic of the dihydrate:

| Angle [°2θ] | d-value CuKα [Å] | Intensity |
|---|---|---|
| 9.44 | 9.36 | very strong |
| 10.93 | 8.09 | strong |
| 16.70 | 5.31 | strong |
| 17.38 | 5.10 | strong |
| 17.62 | 5.03 | strong |
| 17.99 | 4.93 | very strong |
| 20.01 | 4.43 | strong |
| 21.95 | 4.05 | strong |
| 22.66 | 3.92 | strong |
| 24.11 | 3.69 | strong |
| 24.34 | 3.65 | strong |
| 24.64 | 3.61 | strong |
| 25.27 | 3.52 | very strong |
| 25.93 | 3.43 | strong |

Karl-Fischer-titration showed 5.5% (w/w) of water which is equivalent to the theoretical value.

## Claims

1. A hydrated salt of N,N'-diacetylcystine, wherein the hydrated salt has the formula 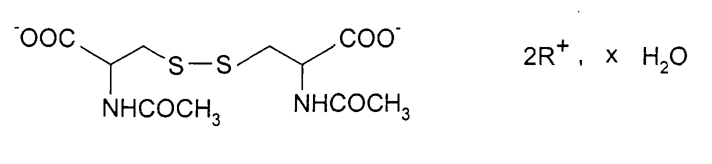 in which
the N,N'-diacetylcystine is the D-, L-, or meso form, or any mixture thereof,
R⁺ is the protonated form of lysine, and x is 2, designating the dihydrate.

2. A hydrated salt as claimed in claim 1, in which the N,N'-diacetylcystine is the L-form.

3. A hydrated salt as claimed in claim 1, wherein said salt is di-L-lysinium-N,N'-diacetyl-L-cystinate dihydrate.

4. A process for the preparation of a hydrated salt of N,N'-diacetylcystine, wherein the hydrated salt has the formula 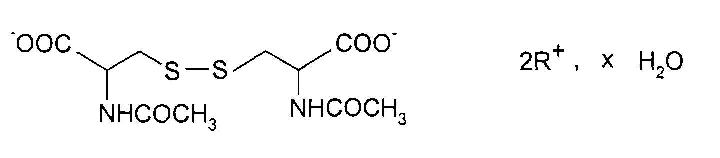 in which
the N,N'-diacetylcystine is the D-, L-, or meso form, or any mixture thereof,
R⁺ is the protonated form of lysine, and x is 2, designating the dihydrate,
comprising
(a) providing a first solution comprising i) N-acetyl cysteine, ii) lysine, or the protonated forms thereof, iii) a solvent, wherein the solvent is water or alcohol, and iv) optionally a catalytic amount of an alkali metal hydroxide;
(b) applying an oxidant to the solution; and
(c) crystallising by adding ethanol, or a mixture of ethanol and water.

5. A therapeutic composition comprising as active ingredient a hydrated salt as claimed in any one of claims 1-3.

6. A therapeutic composition as claimed in claim 5, formulated for administration by inhalation from a dry powder inhaler.

7. Use of a hydrated salt as claimed in any one of claims 1-3, in the preparation of a medicament with immunomodulating activity.

8. Use of a hydrated salt as claimed in any one of claims 1-3, in the preparation of a medicament for the treatment of chronic bronchitis.

9. Use of a hydrated salt as claimed in any one of claims 1-3, in the preparation of a medicament for the treatment of malignant diseases.

10. Use of a hydrated salt as claimed in any one of claims 1-3, in the preparation of a medicament for the treatment of chronic infections.

11. Use of a hydrated salt as claimed in any one of claims 1-3, in the preparation of a medicament for the treatment of chronic hepatitis B and/or C infections.

## Patentansprüche

1. Hydratisiertes N,N'-Diacetylcystin-Salz mit der Formel 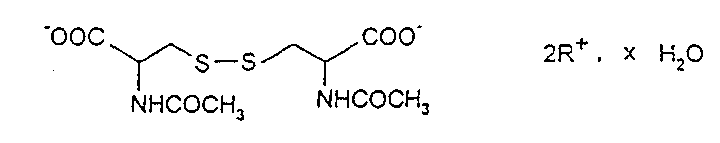 worin
das N,N'-Diacetylcystin in der D-, L- oder meso-Form oder in Form eines Gemischs davon vorliegt, R⁺ die protonierte Form von Lysin darstellt und x für 2 steht und somit das Dihydrat kennzeichnet.

2. Hydratisiertes Salz nach Anspruch 1, in dem das N,N'-Diacetylcystin in der L-Form vorliegt.

3. Hydratisiertes Salz nach Anspruch 1, bei dem es sich um Di-L-lysinium-N,N'-diacetyl-L-cystinat-dihydrat handelt.

4. Verfahren zur Herstellung eines hydratisierten N,N'-Diacetylcystin-Salzes mit der Formel 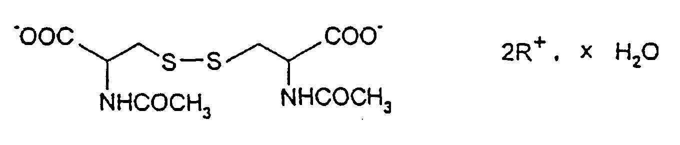 worin
das N,N'-Diacetylcystin in der D-, L- oder meso-Form oder in Form eines Gemischs davon vorliegt, R⁺ die protonierte Form von Lysin darstellt und x für 2 steht und somit das Dihydrat kennzeichnet, bei dem man
(a) eine erste Lösung bereitstellt, die i) N-Acetylcystein, ii) Lysin oder dessen protonierte Formen, iii) ein Lösungsmittel, bei dem es sich um Wasser oder Alkohol handelt, und iv) gegebenenfalls eine katalytische Menge eines Alkalihydroxids enthält;
(b) der Lösung ein Oxidationsmittel zusetzt; und
(c) durch Zusatz von Ethanol oder eines Gemischs aus Ethanol und Wasser kristallisiert.

5. Therapeutische Zusammensetzung, die als aktiven Bestandteil ein hydratisiertes Salz nach einem der Ansprüche 1-3 enthält.

6. Therapeutische Zusammensetzung nach Anspruch 5, die für die Verabreichung durch Inhalation aus einem Trockenpulverinhalator formuliert ist.

7. Verwendung eines hydratisierten Salzes nach einem der Ansprüche 1-3 bei der Herstellung eines Arzneimittels mit immunmodulierender Wirkung.

8. Verwendung eines hydratisierten Salzes nach einem der Ansprüche 1-3 bei der Herstellung eines Arzneimittels zur Behandlung von chronischer Bronchitis.

9. Verwendung eines hydratisierten Salzes nach einem der Ansprüche 1-3 bei der Herstellung eines Arzneimittels zur Behandlung von malignen Erkrankungen.

10. Verwendung eines hydratisierten Salzes nach seinem der Ansprüche 1-3 bei der Herstellung eines Arzneimittels zur Behandlung von chronischen Infektionen.

11. Verwendung eines hydratisierten Salzes nach einem der Ansprüche 1-3 bei der Herstellung eines Arzneimittels zur Behandlung von chronischen Hepatitis-B- und/oder Hepatitis-C-Infektionen.

## Revendications

1. Sel hydraté de N,N'-diacétylcystine, le sel hydraté étant de formule 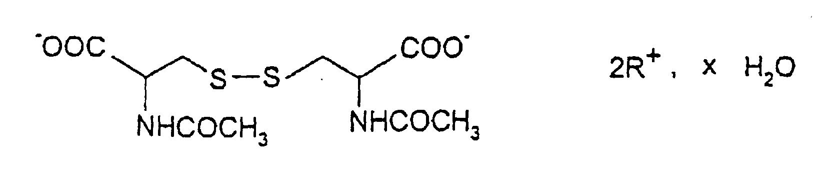 dans laquelle
la N,N'-diacétylcystine est la forme D, L ou méso, ou un mélange quelconque de celles-ci,
R⁺ est la forme protonée de la lysine, et x vaut 2, désignant le dihydrate.

2. Sel hydraté selon la revendication 1, dans lequel la N,N'-diacétylcystine est la forme L.

3. Sel hydraté selon la revendication 1, ledit sel étant le dihydrate de L-lysinium-N,N'-diacétyl-L-cystinate.

4. Procédé de préparation d'un sel hydraté de N,N'-diacétylcystine, dans lequel le sel hydraté est de formule 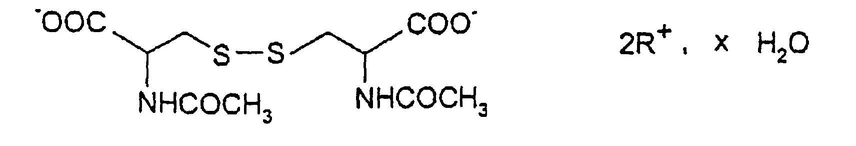 dans laquelle
la N,N'-diacétylcystine est la forme D, L ou méso, ou un mélange quelconque de celles-ci,
R⁺ est la forme protonée de la lysine, et x vaut 2, désignant le dihydrate,
comprenant
(a) la fourniture d'une première solution comprenant i) de la N-acétylcystéine, ii) de la lysine ou ses formes protonées, iii) un solvant, le solvant étant l'eau ou un alcool, et iv) éventuellement une quantité catalytique d'un hydroxyde de métal alcalin ;
(b) l'application d'un oxydant à la solution ; et
(c) la cristallisation en ajoutant de l'éthanol, ou un mélange d'éthanol et d'eau.

5. Composition thérapeutique comprenant, en tant qu'ingrédient actif, un sel hydraté selon l'une quelconque des revendications 1 à 3.

6. Composition thérapeutique selon la revendication 5, formulée en vue d'une administration à partir d'un inhalateur à poudre sèche.

7. Utilisation d'un sel hydraté selon l'une quelconque des revendications 1 à 3, pour la préparation d'un médicament ayant une activité immunomodulatrice.

8. Utilisation d'un sel hydraté selon l'une quelconque des revendications 1 à 3, pour la préparation d'un médicament destiné au traitement d'une bronchite chronique.

9. Utilisation d'un sel hydraté selon l'une quelconque des revendications 1 à 3, pour la préparation d'un médicament destiné au traitement de maladies malignes.

10. Utilisation d'un sel hydraté selon l'une quelconque des revendications 1 à 3, pour la préparation d'un médicament destiné au traitement d'infections chroniques.

11. Utilisation d'un sel hydraté selon l'une quelconque des revendications 1 à 3, pour la préparation d'un médicament destiné au traitement d'infections chroniques d'hépatite B et/ou C.
